# EUROPÄISCHE PATENTSCHRIFT

(11) **EP 3 957 178 B1**
(45) Veröffentlichungstag und Bekanntmachung des Hinweises auf die Patenterteilung: **15.10.2025**
(21) Anmeldenummer: 20192008.9
(22) Anmeldetag: 20.08.2020
(51) Int. Cl.: A01N 65/36, A01N 31/14, A01N 25/02, A01N 25/30, A01N 25/34, A01P 1/00, A61K 8/9789, A61K 8/34, A61K 8/46, A61Q 19/10

(54) **ZUSAMMENSETZUNGEN ZUR REINIGUNG VON HAUT UND/ODER HÄNDEN**
COMPOSITION FOR CLEANING SKIN AND/OR HANDS
COMPOSITIONS DESTINÉES AU NETTOYAGE DE LA PEAU ET/OU DES MAINS

(43) Veröffentlichungstag der Anmeldung: 23.02.2022
(73) Patentinhaber: Menno Chemie-Vertrieb GmbH, 22850 Norderstedt (DE)
(72) Erfinder: NEVERMANN, Jan, 22397 Hamburg (DE); ZERLING, Wolfgang, 24568 Kaltenkirchen (DE)
(74) Vertreter: Becker, Eberhard

(56) Entgegenhaltungen:
- CN-A- 104 116 659
- CN-A- 104 116 664
- CN-A- 111 286 425
- DE-A1- 102005 056 289
- DE-A1- 19 927 936
- GB-A- 2 010 892
- ODENDAAL ISOBEL: "When Life Hands You Lemons... Get Cleaning!", ONBOARDONLINE, 10 August 2015 (2015-08-10), pages 1 - 13, XP055773552, Retrieved from the Internet <URL:https://www.onboardonline.com/yacht-crew/operations/interior/when-life-hands-you-lemons...-get-cleaning/#:~:text=Remove%20fruit%2Fberry%20stains%20from,much%20easier%20than%20soap%20alone.> [retrieved on 20210208]
- .: "Maxi-Grabbs", 23 February 2019 (2019-02-23), Amazon Webseite, pages 1 - 3, XP055773572, Retrieved from the Internet <URL:https://www.amazon.de/Handreinigungst%C3%BCcher-Maxi-Grabbs-90-St%C3%BCck-Werkstatt/dp/B07P2TLC7Z/ref=sr_1_101?dchild=1&keywords=TECHNOLIT&qid=1612802128&sr=8-101> [retrieved on 20210208]
- DATABASE GNPD [online] MINTEL; 7 April 2014 (2014-04-07), ANONYMOUS: "Antibacterial Handwash with Lime & Lemon", XP055833637, retrieved from https://www.gnpd.com/sinatra/recordpage/2338270/ Database accession no. 2338270
- DATABASE GNPD [online] MINTEL; 8 August 2018 (2018-08-08), ANONYMOUS: "Germ Protection Hand Wash", XP93099385, retrieved from https://www.gnpd.com/sinatra/recordpage/5885873/ Database accession no. 5885873
- CASSIDAY LAURA: "Surfactants and Skin", INTERNATIONAL NEWS ON FATS, OILS, AND RELATED MATERIALS (INFORM), 1 January 2016 (2016-01-01), U.S.A., pages 694 - 600, XP93099604, Retrieved from the Internet <URL:https://www.aocs.org/documents/InformPDF/Inform_JAN_2016.pdf> [retrieved on 20231109]
- EFFENDY AND MAIBACH: "Surfactants and experimental irritant contact dermatitis", CONTACT DERMATITIS, vol. 33, 1 January 1995 (1995-01-01), pages 217 - 225, XP055127310, DOI: 10.1111/j.1600-0536.1995.tb00470.x

## Beschreibung

Die vorliegende Erfindung betrifft ökologisch günstige und in ihrer Reinigungswirkung verbesserte Mittel zur Reinigung der Haut und/oder der Hände. Der Anwendungsbereich der vorliegenden Erfindung liegt daher insbesondere im Bereich der hygienischen Entfernung von organischen und/oder anorganischen Verschmutzungen von der Haut, die insbesondere bei Personen auftreten, die in der Landwirtschaft, im Gartenbau, in der Gemüseproduktion, der Lebensmittelverarbeitung pflanzlicher Produkte, oder in Meristem- oder Pflanzenzuchtbetrieben tätig sind.

Das erfindungsgemäße Mittel basiert auf synergistisch wirksamen Gemischen von Zitrusfruchtsäften oder Zitrusfruchtsaftkonzentraten der Familie der Rutaceae, Phenoxyethanol und Tensiden. Die erfindungsgemäße Zusammensetzung ist sehr effektiv in ihrer Wirksamkeit, zeichnet sich aber auch durch ökologisch und dermatologisch günstige Eigenschaften aus.

### Stand der Technik

Personen die in der Landwirtschaft, im Gartenbau, in der Gemüseproduktion, in der Lebensmittelverarbeitung pflanzlicher Produkte oder in Meristem- oder Pflanzenzuchtbetrieben tätig sind, haben besondere Anforderungen an Reinigungsmittel für Haut und Hände. Durch den häufigen Kontakt mit farbigen Anschmutzungen sind die Hände in besonderer Weise stark belastet. So sind in der chlorophyllhaltigen Schmutzfracht nicht nur Pflanzenfarbstoffe enthalten, sondern auch Reste von Pflanzenschutzmitteln und sonstige Hilfsstoffe (Dünger, Herbiziden, Bakteriziden, Keimhemmungsmitteln, Fungiziden usw.) die in undefinierbarer Weise auf das Stratum corneum der Haut einwirken.

Das Stratum corneum besteht vereinfacht aus zwei Komponenten, den Korneozyten (proteinreiche Hornzelllen) und einer lipophilen Interzellularsubstanz. Zusammen mit Fett und Wasser bilden diese die Epidermis, die schützende Schicht der Haut. Bei dem ständigen Kontakt mit der Umgebung, wird insbesondere die Haut der Hände stark beansprucht.

Ein besonders gut sichtbares Beispiel für die intensive Wechselwirkung zwischen Pflanze und Haut wird durch das Ausgeizen der Tomatenpflanzen offenkundig. Als Ausgeizen wird das Abbrechen bestimmter Triebe bezeichnet, die der wachsenden Frucht Teile der Nährsalze und des Wassers entziehen. Ein solcher Trieb wird in der Fachsprache als Geiz bezeichnet und seine Entfernung demzufolge als Ausgeizen.

Chlorophyllhaltige Farbstoffe dringen dabei tief in die oberen Hautschichten ein und sind nur unter Zuhilfenahme aggressiver Hilfsmittel zu entfernen, wobei es durch die arbeitsbedingt fortgesetzten Wiederholungen zu starken Belastungen der Epidermis kommt. Dies kann zu krankhaften Veränderungen der Hautoberfläche führen.

Benötigt wird also ein Reinigungsmittel für die Haut oder die Hände, dass zwar effektiv ist, aber andererseits auch eine gute Verträglichkeit, insbesondere bei häufiger Benutzung aufweist.

Daher wurde nach einer dermatologisch und ökologisch günstigen Formulierung gesucht, die hautfreundlich ist und den Anforderungen der Betroffenen genügt, andererseits aber auch effektiv in der Reinigungswirkung und auch stark genug ist, die anfallenden Reste aus der Verwendung der Neben- und Hilfsprodukte der Zuchtbetriebe gründlich zu entfernen.

Eine Lösung dieser Probleme war bislang nicht in Sicht. Diverse tensidische Zubereitungen konnten keinen ausreichenden Effekt erzielen.

Ein weiteres Problem mit Reinigungsformulierungen gemäß dem Stand der Technik ist, dass diese Alkohole und/oder Parfüms enthalten, die in Wechselwirkung mit der Pflanze und pflanzlichen Produkten (Obst, Gemüse usw.) zu Rückständen und/oder geruchlichen Beeinträchtigungen führen und/oder ihrerseits rückstandsrelevante Betrachtungen nach sich ziehen. Sowohl Rückständen und/oder geruchliche Beeinträchtigungen können in der Verarbeitung oder dem Verkauf des pflanzlichen Produktes ein Ausschlusskriterium sein für dessen Verkaufseignung.

Darüber hinaus sind zunehmend der Nachhaltigkeitsgedanke und die Eignung für eine ökologische Produktionskette maßgeblich. Ziel ist es dabei nur noch ein Minimum an synthetischen, chemischen Komponenten in derartigen Produkten zur Anwendung zu bringen.

Von besonderer Bedeutung ist auch, dass insbesondere Pflanzen, Pflanzenteile, Saatgut, Pollen und insbesondere Pflanzensaft auch für die Übertragung von phytopathogenen bakteriellen, pilzlichen sowie viralen Schaderregern maßgeblich mitverantwortlich sind. Eine derartige Verschmutzung muss von der Haut wirksam entfernt werden, um eine Weiterübertragung zu verhindern. Solche Pathogene können ein zusätzliches Erschwernis für eine Händedesinfektion darstellen.

Deswegen ist eine zielgerichtete hygienische Händewaschung und Händereinigung von essentieller Bedeutung. Pflanzenrückstände, Pflanzenteile, Saatgut, Pollen und insbesondere Pflanzensaft stellen gegenüber der menschlichen Haut eine besonders stark adsorptive, spezifische und hartnäckige Verschmutzung dar, die sich besonders schwer und mit den bisher üblichen Tensiden, Waschmitteln, und Handseifen nicht ausreichend gründlich entfernen lässt. Verbleiben diese pflanzenspezifischen Verschmutzungen auf der menschlichen Haut, ist damit auch eine folgende Desinfektion nur eingeschränkt möglich und durch die pflanzenspezifischen Verschmutzungen nur stark vermindert möglich.

Eine nicht ausreichend durchführbare hygienische Händewaschung oder Händereinigung bedingt dann ein Risiko der Übertragung von Rückständen (Dünger, Herbiziden, Bakteriziden, Keimhemmungsmitteln, Fungiziden usw.), aber auch von alkoholischen und chemisch, synthetischen Komponenten (Parfüms) und letztendlich auch von Mikroorganismen in Folge der nicht ausreichenden, anschließenden Händedesinfektion.

Aufgabe der vorliegenden Erfindung ist es daher, ein ökologisch und dermatologisch günstiges Mittel, aber gleichzeitig effektives Mittel zur Reinigung und Dekontamination der Haut und/oder der Hände bereitzustellen.

DE 199 27 936 Al offenbart Desinfektionsmittelzusammensetzungen enthaltend synergistisch wirksame Kombinationen von Fruchtsäften der Zitrone und/oder der Orange und/oder der *Citrus grandis* in Verbindungen mit Alkylsulfonaten und/oder Alkylarylsulfonaten bzw.- sulfaten, oder Fettalkoholethersulfaten.

Weiterhin werden in "Maxi-Grabbs", 23. Februar 2019, Seiten 1-3, feuchte Handreinigungstücher beschrieben, die ein Tuch umfassen, welches mit einer Zusammensetzung enthaltend Phenoxyethanol, Öl aus der Schale süßer Orangen, kationische und nichtionische Tenside, weitere Konservierungsmittel, Lösungsvermittler und Wasser getränkt ist. Wobei die Tücher zur Reinigung der Haut von Fetten, Schmier- und Klebstoffen, Ölen, Teer, Asphalt, Tinte, Wachs, Kohlenstoff und Grasflecken geeignet sind.

In der DATABASE GNDP MINTEL, 7. April 2014, "Antibaterial Handwash with Lime & Lemon", Database Zugangsnr.: 2338270 wird ein gebrauchsfertiges antimikrobielles Handwaschmittel beschrieben, das unter anderem Saft der Mexikanischen Limette, Zitronensaft, Natriumlaurethsulfat, Phenoxyethanol und Wasser enthält.

Darüber hinaus wird in der DATABASE GNDP MINTEL, 8. August 2018, "Germ Production Hand Wash", Database Zugangsnr.: 5885873 ein gebrauchsfertiges antimikrobielles Handwaschmittel beschrieben, das unter anderem Orangensaft, Zitronensaft, Natriumlaurethsulfat, Phenoxyethanol und Wasser enthält.

### Beschreibung der vorliegenden Erfindung

Diese Aufgaben werden durch eine Zusammensetzung gelöst, welche einen Zitronensaft oder Zitronensaftkonzentrat, oder einer Mischung von zwei oder mehreren Zitrusfruchtsäften oder Zitrusfruchtsaftkonzentraten aus der Familie der Rutaceae, Phenoxyethanol, mindestens ein anionisches Tensid und Wasser und/oder ein anderes Lösungsmittel umfasst. Überraschend wurde nämlich erfindungsgemäß festgestellt, dass Gemische aus Tensiden in Verbindung mit Phenoxyethanol und dem Zusatz von natürlichem Zitrusfruchtsaft auf der Haut eine herausragende Reinigungsleistung bei gleichzeitiger Hautpflege entfalten. Durch die Verwendung von Säften, die für den menschlichen Genuss vorgesehen sind, werden die günstigen ökologischen und toxikologischen Eigenschaften besonders hervorgehoben.

Die vorliegende Erfindung stellt somit eine Zusammensetzung zur Reinigung von Haut und/oder Händen bereit, welche sich als besonders vorteilhaft zum Entfernen der in Rede stehenden Verschmutzungen von der Haut, beispielsweise von den Händen, erwiesen hat.

Die erfindungsgemäße Zusammensetzung ist in Anspruch 1 beschrieben. Der Umfang der Erfindung und damit der Schutzumfang wird durch die beigefügten Ansprüche definiert. Insbesondere sind im Folgenden genannte Methoden oder Verfahren mit therapeutischen oder prophylaktischen Effekten nicht als Teil der Erfindung zu verstehen. In solchen Fällen ist die Erfindung als auf die Zusammensetzung zur Verwendung in einer solchen Methode bzw. einem solchen Verfahren beschränkt aufzufassen.

Die erfindungsgemäße Zusammensetzung (hierin auch als Mittel bezeichnet) zeichnet sich durch eine vorteilhafte Wirkung bei der Entfernung von organischen und/oder anorganischen Verschmutzungen von der Haut aus. Die erfindungsgemäße Zusammensetzung wird deshalb insbesondere zum Entfernen von Chlorophyll und/oder andere pflanzliche Farbstoffe enthaltenden Verschmutzung und/oder von Pflanzensäften/-extrakten eingesetzt. Ebenso kann die erfindungsgemäße Zusammensetzung zum Entfernen von Phytopathogenen, Düngerrückständen, Herbiziden, Bakteriziden, Fungiziden, Virozide und/oder Keimhemmungsmitteln von der Haut eingesetzt werden.

Die erfindungsgemäßen Zusammensetzungen zeichnen sich durch hohe, aber gleichzeitig schonende Reinigungs- und Dekontaminationswirkung aus. Die erfindungsgemäßen Zusammensetzungen führen, im Vergleich zu konventionellen Mitteln, in deutlich kürzerer Zeit zum gewünschten Erfolg.

Das erfindungsgemäße Mittel umfasst wenigstens einen Zitronensaft oder Zitronensaftkonzentrat, oder einer Mischung von zwei oder mehreren Zitrusfruchtsäften oder Zitrusfruchtsaftkonzentraten. Bei den Zitrusfruchtsäften oder Zitrusfruchtsaftkonzentraten handelt es sich erfindungsgemäß vorzugsweise um Zitrusfruchtsäfte ausgewählt aus der Gruppe, bestehend aus Zitronensaft, Orangensaft, Pampelmusensaft, Grapefruitsaft, Mandarinensaft, Limettensaft, Yuzusaft, Kabosusaft, Sudachisaft, Shiikuwashasaft oder Gemischen daraus. Der oder die Zitrusfruchtsäfte sind also natürlichen Ursprungs. Die Zitrusfruchtsäfte können von Pulpe befreit sein, d.h. sie enthalten weniger als 2 Gew.-%, insbesondere weniger als 1 Gew.-% Pulpe. Die Pulpe kann beispielsweise durch Filtration, Absieben oder Sedimentieren der Rohsäfte entfernt werden.

Pulpe im Sinne der vorliegenden Erfindung meint langfaserige, feste Bestandteile des Fruchtfleisches, die beispielsweise beim Auspressen der Zitrusfrüchte neben dem Fruchtsaft erhalten werden. Langfaserige Bestandteile im Sinne der vorliegenden Erfindung sind vorzugsweise solche mit einer Länge von mindestens 0,5 mm, noch mehr bevorzugt von mindestens 1 mm.

Die erfindungsgemäß eingesetzten Zitrusfruchtsäfte, enthalten in der Regel 3 bis 8 Gew.-%, vorzugsweise 4 bis 7 Gew.-%, Zitronensäure und weisen vorzugsweise einen pH-Wert im Bereich von 1 bis 5, noch mehr bevorzugt im Bereich von 2 bis 4 auf. Der mittlere pH-Wert kann beispielsweise 3 betragen.

Als Zitrusfruchtsäfte für die erfindungsgemäße Zusammensetzung werden vorzugsweise solche Säfte ausgewählt, die aus frischen Früchten gewonnen wurden und gegebenenfalls mit einem Enzym, beispielsweise Pektinase, behandelt wurden, um den Fruchtsaft aufzuklären. Vorzugsweise beträgt die Transmission bei 650 nm der so erhaltenen Säfte, bei 8° Brix, mindestens 80 %, noch mehr bevorzugt mindestens 90 %. Die Zitrusfruchtsäfte oder deren Konzentrate können auch mittels einer geeigneten, vorzugsweise einer physikalischen Maßnahme haltbar gemacht worden sein. Beispielhaft seien sterilisieren oder pasteurisieren genannt.

Erfindungsgemäß nicht ausgeschlossen sein soll auch, dass den Zitrusfruchtsäften eine für den Mensch unschädliche Säure und/oder in der Lebensmittelindustrie zugelassene Säure zugesetzt wird. Solche Säuren schließen beispielsweise Zitronensäure und Äpfelsäure ein, sind aber nicht hierauf beschränkt.

Neben der natürlichen Zitronensäure enthalten die Zitrusfrüchte Terpene, wobei die Hauptkomponente (70 bis 90 % der Zitrusöle) das R(+)Limonen ist. Zu den weiteren typischen Aromastoffen der Zitrusfrüchte gehören die Aldehyde Octanal, Nonanal sowie α-Pinen, Valencen, α- und β-Sinensal und auch Neral und Geranial. Derartige Aromastoffe lassen sich überwiegend aus der Schale von Zitrusfrüchten isolieren. Darüber hinaus sind Zitrusfruchtsäfte reich an Vitamin C, das sich positiv auf die Hautpflege auswirkt und eine antioxidative Eigenschaft aufweist.

Wesentlich ist daher, dass erfindungsgemäß der gesamte Zitrusfruchtsaft, der unter anderem auch für den menschlichen Genuss geeignet ist, eingesetzt wird. Diese Fruchtsäfte können einen hohen Wassergehalt, beispielsweise im Bereich von 75 bis 95 Gew.-%, aufweisen. Deshalb ist es erfindungsgemäß auch vorgesehen, dass die Zitrusfruchtsäfte aufkonzentriert sein können.

Erfindungsgemäße Zitrussaftkonzentrate können 1-10-fache Konzentrate, vorzugsweise 2-8-fache, noch mehr bevorzugt 3-7-fache Konzentrate des oder der ursprünglichen Zitrusfruchtsäfte sein. Die Gesamtsäuremasse solcher Konzentrate kann vorzugsweise im Bereich von 75 bis 770 g pro Liter, noch mehr bevorzugt im Bereich von 150 bis 620 g pro Liter liegen. Sie kann beispielsweise 500 g pro Liter betragen. Die Dichte solcher Konzentrate, bei 20 °C, kann vorzugsweise im Bereich von 0,8 bis 1,6, noch mehr bevorzugt im Bereich von 1 bis 1,4 liegen.

Ein Zitrusfruchtsaft oder dessen Konzentrat zum Einsatz in der erfindungsgemäßen Zusammensetzung kann beispielsweise hergestellt werden durch ein Verfahren, welches die folgenden Schritte umfasst: Auspressen der vorzugsweise gewaschenen Zitrusfrüchte mit Hilfe einer Saftpresse und gegebenenfalls Aufkonzentrieren des so erhaltenen Zitrusfruchtsaftes. Das Aufkonzentrieren erfolgt in an sich bekannter Weise auf das gewünschte, wie vorstehend spezifizierte Volumen, um so ein Konzentrat des Zitrusfruchtsaftes zu erhalten.

Nach dem Auspressen in einer Saftpresse kann der so erhaltene Saft, falls erforderlich, beispielsweise in einen Zyklonabscheider eingespeist werden, um Samen im Saft zu entfernen. Danach kann der Saft beispielsweise in einen Abscheider eingespeist werden, um die Pulpe abzutrennen, falls dies gewünscht ist.

Eine optionale Sterilisation kann beispielsweise unter folgenden Bedingungen erfolgen: Vorwärmen des Saftes oder dessen Konzentrats auf eine Temperatur im Bereich von 55 bis 60 °C zum Entgasen, anschließend Erhöhen und der Temperatur auf einen Bereich von 75 bis 85 °C und Halten dieser Temperatur für vorzugsweise 20 bis 60 Sekunden zur Sterilisation und Inaktivierung von Enzymen. Danach wird die Flüssigkeit auf unter 15 °C abgekühlt.

Dem Saft oder dem Konzentrat kann, falls gewünscht eine für den Mensch unschädliche Säure, in der Lebensmittelindustrie zugelassene Säure zugesetzt werden. Diese Säure kann auch Zitronensäure sein. Der Zusatz einer oder mehrerer solcher Säuren beträgt aber vorzugsweise maximal 1 Gew.-%, noch mehr bevorzugt maximal 0,5 Gew.-% oder maximal 0,3 Gew.-%, bezogen auf den aus den Zitrusfrüchten erhaltenen Rohsaft.

Zur Standardisierung wird der erhaltene Zitrusfruchtsaft einem Säuretest unterzogen und dessen Gehalt an Zitronensäure bestimmt. Der Gehalt an Zitronensäure im Saft vor dessen wahlweiser Aufkonzentrierung sollte im Bereich von 3 bis 8 Gew.-%, vorzugsweise im Bereich von 4 bis 7 Gew.-%, liegen.

Die Gesamtsäuremasse der hieraus erhältlichen Konzentrate sollte vorzugsweise im Bereich von 75 bis 770 g pro Liter, noch mehr bevorzugt im Bereich von 150 bis 620 g pro Liter liegen. Die Dichte solcher Konzentrate, bei 20 °C, sollte vorzugsweise im Bereich von 0,8 bis 1,6, noch mehr bevorzugt im Bereich von 1 bis 1,4 liegen. Der pH-Wert der Konzentrate sollte vorzugweise im Bereich von 1 bis 5, noch mehr bevorzugt im Bereich von 2 bis 4 liegen.

Geklärte Konzentrate oder Säfte sollten, bei 8° Brix, vorzugsweise eine Transmission bei 650 nm von mindestens 80 %, noch mehr bevorzugt von mindestens 90 %, aufweisen.

Der Anteil des Zitrusfruchtsafts oder des Zitrusfruchtsaftkonzentrats im erfindungsgemäßen Mittel 5 bis 30 Gew.-%, bevorzugt 7 bis 25 Gew.-%, und noch bevorzugter 10 bis 20 Gew.-%.

Der Zitronensaft ist natürlichen Ursprungs. Der Zitronensaft enthält vorzugsweise 3 bis 8 Gew.-%, noch mehr bevorzugt 4 bis 7 Gew.-%, Zitronensäure und weist vorzugsweise einen pH-Wert im Bereich von 1 bis 5, noch mehr bevorzugt im Bereich von 2 bis 4 auf. Der Zitronensaft kann von Pulpe befreit sein. Erfindungsgemäß bevorzugte Zitronensaftkonzentrate sind 1-10-fache Konzentrate, vorzugsweise 2-8-fache, noch mehr bevorzugt 3-7-fache Konzentrate.

Der Zitronensaft oder dessen Konzentrat kann wie vorstehend beschrieben hergestellt worden sein oder nach diesem Verfahren erhältlich sein.

Die Gesamtsäuremasse des Zitronensaft-Konzentrats liegt vorzugsweise im Bereich von 75 bis 770 g pro Liter, noch mehr bevorzugt im Bereich von 150 bis 620 g pro Liter. Die Dichte solcher Konzentrate kann, bei 20 °C, vorzugsweise im Bereich von 0,8 bis 1,6, noch mehr bevorzugt im Bereich von 1 bis 1,4 liegen. Der pH-Wert der Konzentrate sollte vorzugsweise im Bereich von 1 bis 5, noch mehr bevorzugt im Bereich von 2 bis 4 liegen.

Geklärte Zitronensaftkonzentrate oder Zitronensäfte sollten, bei 8° Brix, vorzugsweise eine Transmission bei 650 nm von mindestens 80 %, noch mehr bevorzugt von mindestens 90 %, aufweisen.

Der Anteil des Zitronensafts oder des Zitronensaftkonzentrats im erfindungsgemäßen Mittel beträgt 5 bis 30 Gew.-%, vorzugsweise 7 bis 25 Gew.-%, noch bevorzugter 10 bis 20 Gew.-% .

Für den Fall, dass die erfindungsgemäße Zusammensetzung als Zitrusfruchtsaft oder Zitrusfruchtsaftkonzentrat ein Gemisch von zwei oder mehr Zitrusfruchtsäften enthält, ist in einer solchen Mischung der Zitronensaft oder das Zitronensaftkonzentrat enthalten.

Der Anteil des vorstehend beschriebenen Gemisches von Zitrusfruchtsäften oder von Zitrusfruchtsaftkonzentraten im erfindungsgemäßen Mittel beträgt 5 bis 30 Gew.-%, vorzugsweise 7 bis 25 Gew.-%, noch bevorzugter 10 bis 20 Gew.-% .

Die erfindungsgemäße Zusammensetzung umfasst weiterhin Phenoxyethanol. Es handelt sich hierbei um einen Ether des Phenols mit Ethylenglycol der Summenformel C₈H₁₀0₂. Der Anteil des Phenoxyethanols im erfindungsgemäßen Mittel beträgt 0,5 bis 10 Gew.-%, vorzugsweise 1 bis 8 Gew.-%, noch bevorzugter 1,5 bis 6 Gew.-%.

Das Gewichtsverhältnis von Zitrusfruchtsaft oder Zitrusfruchtsaftkonzentrat, vorzugsweise Zitronensaft oder einer Zitrusfruchtsaftmischung mit Zitronensaft, zu Phenoxyethanol liegt vorzugsweise im Bereich von 1:0,1 bis 1:0,8, noch mehr bevorzugt im Bereich von 1:0,3 bis 1:0,6.

Es hat sich überraschenderweise gezeigt, dass durch den kombinatorischen Einsatz eines Zitrusfruchtsafts, vorzugsweise von Zitronensaft oder einer Zitrusfruchtsaftmischung mit Zitronensaft, mit Phenoxyethanol hervorragende Reinigungseffekte der Haut erzielt werden können.

Die erfindungsgemäße Zusammensetzung enthält mindestens ein anionisches Tensid mit einem Gesamtanteil von 2 bis 60 Gew.-%, bezogen auf die Gesamtzusammensetzung. Anionische Tenside sind Tenside, die eine negativ geladene funktionelle Gruppe aufweisen. Wie alle Tenside sind auch die anionischen Tenside aus einem polaren und einem unpolaren Teil aufgebaut. Als unpolarer Teil kann ein Alkylrest fungieren. Die polare, funktionelle Gruppe kann beispielsweise eine Carboxylatgruppe (-COO⁻), eine Sulfonatgruppe (-SO₃⁻) oder eine Sulfatgruppe (-SO₄²⁻) sein.

Das oder die anionischen Tenside sind ausgewählt aus der Gruppe, bestehend aus sekundären Alkansulfonsäuren, sekundären Alkansulfonaten, Alkylethersulfaten, Alkylsulfaten und beliebigen Mischungen davon.

Alkylsulfate sind wasserlösliche Salze oder Säuren der Formel ROSO₃M, wobei R ein C₁₀-C₂₄-Kohlenwasserstoffrest, bevorzugt ein C₁₀-C₂₀-Alkyl- oder Hydroxyalkylrest, besonders bevorzugt ein C₁₂-C₁₈-Alkyl- oder Hydroxyalkylrest ist. M ist Natrium oder Kalium.

Bei den Alkylethersulfaten (diese werden im Stand der Technik auch als Fettalkoholethersulfate, INCI Alkyl Ether Sulfates, bezeichnet) handelt es sich um wasserlösliche Salze oder Säuren der Formel RO(A)ₘSO₃M, wobei R einen unsubstituierten C₈-C₁₈-Alkyl- oder Hydroxyalkylrest, bevorzugt einen C₁₀-C₁₆-Alkyl- oder Hydroxyalkylrest, besonders bevorzugt C₁₂-C₁₄-Alkyl- oder Hydroxyalkylrest darstellt. A ist eine Alkylenoxideinheit, bevorzugt eine Ethylenoxid, m ist eine Zahl von 3 bis 9 (Ethoxylierungsgrad im Bereich von EO 3 bis 9), besonders bevorzugt zwischen ca. 3 und ca. 7 (Ethoxylierungsgrad im Bereich von EO 3 bis 7), und M ist Natrium oder Kalium.
Als Beispiele seien C₁₀- bis C₁₄-Fettalkoholethersulfate genannt wobei der Gehalt an EO 3, 4, 5, 6, 7, 8 oder 9 mol pro mol des Fettalkoholethersulfats beträgt, und in denen M Natrium oder Kalium ist.

Sekundäre Alkansulfonate sind die Salze der sekundären Alkansulfonsäuren. Bei diesen anionischen Tensiden kann die Alkylgruppe entweder gesättigt oder ungesättigt, verzweigt oder linear und gegebenenfalls mit einer Hydroxylgruppe substituiert sein. Die Sulfogruppe kann an einer beliebigen Position der C-Kette stehen, wobei die primären Methylgruppen am Kettenanfang und Kettenende keine Sulfonatgruppen besitzen. Die sekundären Alkansulfonate enthalten lineare primäre und/oder sekundäre Alkylketten mit jeweils 10 bis 20 Kohlenstoffatome und besonders bevorzugt 12 bis 18 Kohlenstoffatomen. Das Kation ist Natrium oder Kalium.

Beispielhafte Alkylreste der vorstehend genannten anionischen Tenside mit 10 bis 13 Kohlenstoffatomen sind die n-Decyl-, die n-Undecyl- und die n-Dodecylgruppe. Primäre oder sekundäre Ketten der Länge (C₈-C₁₈) werden durch folgende Gruppen veranschaulicht: n-Octyl-, 2-Octyl-, n-Nonyl-, 2-Nonyl-, n-Decyl-, 2-Decyl-, n-Undecyl-, 2-Undecyl-, n-Dodecyl-, 2-Docecyl-, n-Tridecyl-, 2-Tridecyl, n-Tetradecyl-, 2-Tetradecyl-, n-Pentadecyl-, 2-Pentadecyl-, n-Hexadecyl-, 2-Hexadecyl-, n-Heptadecyl-, 2-Heptadecyl-, n-Octadecyl- und 2-Octadecylgruppe.

Bei den anionischen Tensiden haben sich die Alkylethersulfate als besonders vorteilhaft erwiesen. Erfindungsgemäß besonders bevorzugte anionische Tenside sind deshalb Alkylethersulfate oder deren Natrium-Salze. Erfindungsgemäss verwendbar sind Alkylethersulfate mit 8 bis 18 Kohlenstoffatomen, höchst bevorzugt mit 12 bis 14 Kohlenstoffatomen, bzw. deren Salze, vorzugsweise deren Natriumsalze.

Das erfindungsgemäß in der Zusammensetzung umfasste anionische Tensid oder das anionische Tensidgemisch, vorzugsweise die bevorzugten sekundären Alkansulfonsäuren, sekundären Alkansulfonate, Alkylethersulfate, und/oder Alkylsulfate, sind in den erfindungsgemäßen Zusammensetzungen einzeln oder in beliebiger Kombination untereinander mit einem Anteil von 2 bis 60 Gew.-%, insbesondere mit einem Anteil von 4 bis 50 Gew.-%, ganz besonders bevorzugt mit einem Anteil von 5 bis 40 Gew.-%, bezogen auf die Gesamtzusammensetzung, umfasst.

Das anionische Tensid ist oder umfasst die anionische Tensidmischung mindestens eines der vorstehend beschriebenen Alkylethersulfate. In den Tensidmischungen vorzugsweise in Kombination mit mindestens einem Alkylsulfat oder mit mindestens einem sekundären Alkansulfonat oder einer sekundären Alkansulfonsäuren. Das Alkylsulfat oder die Alkylsulfate und/oder das sekundäre Alkansulfonat, die sekundären Alkansulfonsäure oder die mehreren sekundären Alkansulfonate und/oder sekundären Alkansulfonsäuren sind wiederum wie vorstehend beschrieben ausgebildet.

In den anionischen Tensidmischungen ist das oder sind die Alkylethersulfate vorzugsweise mit einem Anteil von 1 bis 30 Gew.-%, vorzugsweise mit einem Anteil von 2 bis 20 Gew.-%, ganz besonders bevorzugt mit einem Anteil von 3 bis 15 Gew.-%, bezogen auf die Gesamtzusammensetzung, und das mindestens eine Alkylsulfat oder das mindestens eine sekundäre Alkansulfonat oder die mindestens eine sekundäre Alkansulfonat ist/sind mit einem mit einem Anteil von 1 bis 30 Gew.-%, vorzugsweise mit einem Anteil von 2 bis 20 Gew.-%, ganz besonders bevorzugt mit einem Anteil von 3 bis 15 Gew.-%, bezogen auf die Gesamtzusammensetzung, umfasst.

Die erfindungsgemäße Zusammensetzung umfasst weiterhin Wasser (als Lösungsmittel) und/oder wenigstens ein weiteres Lösungsmittel außer Wasser. Vorzugsweise ist das Lösungsmittel Wasser oder es umfasst Wasser in einem Lösungsmittelgemisch. Andere Lösungsmittel außer Wasser sind dem Fachmann bekannt. Sie umfassen beispielsweise wässerige, gepufferte Lösungen.

Der Anteil des Wassers ist der Rest auf 100 Gew.-% (ad. 100 Gew.-%), bezogen auf die Gesamtzusammensetzung des erfindungsgemäßen Reinigungsmittels. Das heißt die Gew.-%-Anteile der wesentlichen und gegebenenfalls vorhandenen optionalen Bestandteile der Zusammensetzung sind immer so gewählt, dass sich in der Summe 100 Gew.-% ergeben.

Bei dem erfindungsgemäßen Mittel handelt es sich vorzugsweise um eine flüssige, saure Zusammensetzung. Das erfindungsgemäße Mittel weist vorzugsweise einen sauren pH-Wert im Bereich von 4,75 bis 5,75 auf. Der pH-Wert der Zusammensetzung kann beispielsweise 5,5 sein.

Das erfindungsgemäße Mittel umfasst ferner mindestens ein Hydrotropierungsmittel. Das Hydrotropierungsmittel ist oder umfasst Toluolsulfonat und /oder Cumolsulfonat und/oder deren Natrium- oder Kaliumsalze. Das Hydrotropierungsmittel oder die Mischung von Hydrotropierungsmitteln, das Toluolsulfonat und /oder das Cumolsulfonat und/oder deren Natrium- oder Kaliumsalze oder eine Mischung mit Toluolsulfonat und /oder Cumolsulfonat und/oder deren Natrium- oder Kaliumsalze, ist mit einem Anteil von 0,5 bis 3 Gew.-% in der erfindungsgemäßen Zusammensetzung enthalten. Bevorzugter beträgt der optionale Anteil an Hydrotropierungsmittel 1 bis 2,8 Gew.-%, noch mehr bevorzugt 1,2 bis 2,6 Gew.-%, jeweils bezogen auf die Gesamtzusammensetzung.

Das erfindungsgemäße Mittel kann optional auch einen oder mehrere übliche Zusatz- oder Hilfsstoffe enthalten. Geeignete Substanzen und Substanzgemische sind dem Fachmann wohl bekannt und können im Handel bezogen werden. Die Zusatz- und Hilfsstoffe können beispielsweise übliche für den Bestimmungszweck annehmbare Formulierungshilfen, Puffersubstanzen, Gerüstsubstanzen, Konservierungsmittel, Verdickungsmittel, Schaumstabilisatoren, Aufschäumhilfen, Feuchthaltemittel, etc. sein. So kann die erfindungsgemäße Zusammensetzung ein oder mehrere Verdickungsmittel enthalten. Die für diesen Zweck geeigneten Verdickungsmittel sind dem Fachmann grundsätzlich bekannt und im Handel erhältlich. Besonders bevorzugte Verdickungsmittel sind Cellulosen und Cellulose-Derivate. Diese Verdickungsmittel haben sich als vorteilhaft in Kombination mit den übrigen wesentlichen und optionalen Bestandteilen des erfindungsgemäßen Mittels erwiesen. Besonders bevorzugt ist Hydroxypropylmethylcellulose wegen günstiger produktionstechnischer Eigenschaften. Das oder die Verdickungsmittel kann/können erfindungsgemäß entweder einzeln oder in Kombination mit weiteren Zusatz- und Hilfsstoffe im erfindungsgemäßen Mittel umfasst sein. Das oder die Verdickungsmittel ist/sind im erfindungsgemäßen Mittel vorzugsweise mit einem Anteil von 0,05 bis 1 Gew.-%, noch bevorzugter von 0,1 bis 0,8 Gew.-%, noch mehr bevorzugt von 0,15 bis 0,6 Gew.-% enthalten. Der Einsatz eines Verdickungsmittels ist deshalb vorteilhaft, weil so beim Waschprozess die aufgebrachte Menge des Mittels auf den Händen während der Reinigung haften bleibt. Bei einem dünnflüssigen Mittel besteht hingegen die Möglichkeit, beim Aufbringen auf die Haut, einen Teil der Reinigungsflüssigkeit zu verlieren.

Als Feuchthaltemittel werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht zu reduzieren und/oder die Hydratation der Hornschicht positiv zu beeinflussen. Beispielhaft, aber nicht einschränkend, seien Glycerin, Milchsäure und Hyaluronsäure genannt.

Pharmazeutisch und/oder kosmetisch akzeptable Schaumstabilisatoren und Aufschäumhilfen sind dem Fachmann grundsätzlich bekannt. Sie können beispielsweise oder vorzugsweise ausgewählt aus der Gruppe der Aminoxide, insbesondere der Alkyl-Aminoxide. Die Alkyl-Aminoxide enthalten vorzugsweise Alkylreste mit einer Kettenlänge von 14 bis 24 Kohlenstoffatomen. Ein bevorzugter Schaumverstärker ist erfindungsgemäß Cocospropylaminoxid bzw. Cocosfettsäureamidopropyldimethylaminoxid bzw. Cocamido-propylaminoxid bzw. Cocosfettsäureamidopropyldimethylammoniumoxid mit verschiedenen Kettenlängen (CAS Nr. 68155-09-9). Der Einsatz eines Schaumverstärkers ist deshalb vorteilhaft, weil der sich aus dem Mittel bildende Schaum während der Reinigung gut an der Haut haftet und so zu einer gründlichen Reinigung führt.

Gemäß einer erfindungsgemäßen Ausführungsform ist das erfindungsgemäße Mittel frei von Geruchsstoffen und/oder Parfüm. Zusätzlich oder alternativ ist das erfindungsgemäße Mittel frei von Alkohol, vorzugsweise von einwertigen C₁-C₅-Alkoholen. Alkohole meinen im Sinne der vorliegenden Erfindung organische Verbindungen, die eine oder mehrere an unterschiedliche aliphatische Kohlenstoffatome gebundene Hydroxygruppen besitzen. Beispielhaft, aber nicht einschränkend, seien die einwertigen Alkohole Methanol, Ethanol und Propanol genannt.

Weiter alternativ oder zusätzlich zu den Geruchsstoffen, Parfüms und/oder Alkoholen kann die erfindungsgemäße Zusammensetzung frei von solchen Komponenten sein, die rückstandsrelevant an den Händen anhaften können und so über einen Kontakt mit den Händen auf Pflanzen, pflanzliche Produkte und/oder Lebensmittel übertragen werden könnten.

Die Angabe "frei von" bedeutet im Sinne der vorliegenden Offenbarung, dass der Anteil an solchen Bestandteilen an der Gesamtzusammensetzung 0 bis weniger als 0,01 Gew.-% beträgt. Vorzugsweise beträgt die maximal zulässige Höchstgrenze 0,008 Gew.-%, noch mehr bevorzugt 0,006 Gew.-% und noch weiter bevorzugt 0,004 Gew.-%, jeweils bezogen auf das Reinigungsmittel. Höchst bevorzugt ist der Anteil solcher Bestandteile in der Zusammensetzung 0 Gew.-%. Etwaige nicht vermeidbare Verunreinigungen sollen hierbei außer Acht bleiben.

Weiterhin ist es erfindungsgemäß bevorzugt, dass die Komponenten des Mittels so zu wählen sind, dass das Mittel biologisch gut abbaubar und dermatologisch gut verträglich ist. Durch die vorstehend beschriebenen Kombinationen der Inhaltsstoffe hat sich überraschend gezeigt, dass eine herausragende Reinigungsleistung ohne aggressive Hilfsmittel und gleichzeitig ein angenehmes Hautgefühl, bei gleichzeitiger Erhaltung des Säureschutzmantels der Haut, erreicht werden kann. Das erfindungsmäße Mittel ist somit besonders hautverträglich und pflegend, ohne dass zusätzlich Hautrückfetter und/oder hautpflegende Komponenten zugesetzt werden müssen. Der Einsatz von Hautrückfettern und/oder hautpflegenden Komponenten ist aber erfindungsgemäß nicht grundsätzlich ausgeschlossen.

Zusätzlich zu den genannten Bestandteilen, kann ein solches Reinigungsmittel optional einen oder mehrere übliche Zusatz- und Hilfsstoffe enthalten.

Ebenfalls im Umfang der vorliegenden Erfindung mit umfasst sind solche Zusammensetzungen bzw. Mittel, die, mit Ausnahme von üblichen Verunreinigungen, nur aus den vorstehend genannten wesentlichen, und gegebenenfalls einer oder mehreren der optionalen Komponenten, bestehen.

Zusätzlich zu den genannten Bestandteilen, kann eine solche Zusammensetzung optional einen oder mehrere übliche Zusatz- und Hilfsstoffe enthalten.

Ebenfalls im Umfang der vorliegenden Erfindung mit umfasst sind solche Zusammensetzungen bzw. Mittel, die, mit Ausnahme von üblichen Verunreinigungen, nur aus den vorstehend genannten wesentlichen, und gegebenenfalls einer oder mehreren der optionalen Komponenten, bestehen.

Bei dem erfindungsgemäßen Mittel handelt es sich um ein flüssiges Einkomponentenprodukt, das als gebrauchsfertiges Produkt formuliert werden kann, das aber andererseits auch als Produktkonzentrat formuliert werden kann, aus welchem eine verdünnte Gebrauchslösung hergestellt werden kann, die dann vorzugsweise 1 bis 30 Gew.-%, noch mehr bevorzugt 2 bis 20 Gew.-% des Mittels enthalten kann. Die Verdünnung des Mittels zur Gebrauchslösung erfolgt vorzugsweise mit Wasser. Die vorliegende Anmeldung betrifft daher auch die so erhaltene fertige Gebrauchslösung.

Die gebrauchsfertige Wirkstofflösung, unabhängig ob sie aus einen Konzentrat hergestellt wird oder bereits ursprünglich so formuliert ist, ist vorzugsweise entweder als Einreibeprodukt oder als Hand-/Hautwaschmittel formuliert. Ein Einreibeprodukt wird zur Reinigung der Haut / der Hände zunächst in die Haut /Hände eingerieben bzw. auf diesen verrieben und nach einer Einwirkzeit von vorzugsweise 30 Sekunden bis 2 Minuten, mit einem Papiertuch und/oder Textiltuch entfernt, wobei das Tuch auch ein feuchtes bzw. ein, beispielsweise mit Wasser, getränktes Tuch sein kann. Im Unterschied dazu ist ein Hand-/ Hautwaschmittel für ein Abwaschprozedere, beispielsweise am Waschbecken. Ein Hand-/Hautwaschmittel wird zur Reinigung der Haut / der Hände zunächst in die Haut / Hände eingerieben bzw. auf diesen verrieben und nach einer Einwirkzeit von vorzugsweise 30 Sekunden bis 2 Minuten dann unter fließendem Wasser abgewaschen.

Die erfindungsgemäße Zusammensetzung, kann aber auch mit Hilfe eines feuchten kosmetischen und/oder dermatologischen Tuchs eingesetzt werden. Die vorliegende Erfindung umfasst deshalb auch Tücher, welche mit der erfindungsgemäßen Zusammensetzung befeuchtet oder getränkt sind.

Die gebrauchsfertigen feuchten oder getränkten Tücher umfassen somit zwei Komponenten, nämlich ein Tuch, welches aus Materialien wie Papier und/oder unterschiedlichsten Mischungen aus Natur- oder Kunstfasern aufgebaut ist, und eine Tränkungslösung. Die Tränkungslösung ist die erfindungsgemäße Zusammensetzung, sei es in unverdünnter oder in verdünnter Form.

Die Tücher erlauben unter anderem eine effiziente und hautschonende Reinigung der Haut, insbesondere der Hände, besonders auch in der Abwesenheit von (fließendem) Wasser.

Als Ausgangsmaterialen für das trockene Tuch können generell alle organischen und anorganischen Faserstoffe auf natürlicher und synthetischer Basis verwendet werden. Beispielhaft seien Zellstoffmaterialien auf der Basis von Cellulose, Gewebe, beispielsweise Baumwollgewebe, und Vliesgewebe (nonwoven material) genannt. Bei dem Vliesgewebe kann es sich beispielsweise um ein wasserstrahlverfestigtes und/oder wasserstrahlgeprägtes Vlies handeln. Als beispielshafte Ausgangsmaterialien für Gewebe und Vliese seien Viskose, Baumwolle, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Materialien und Fasern zur Herstellung des trockenen Tuches eingesetzt werden, sofern die so erhaltenen Tücher kosmetisch und/oder dermatologisch einsetzbar sind.

Die Dicke des trockenen, also noch nicht befeuchteten oder getränkten, Tuchs beträgt vorzugsweise 0,2 mm bis 1,5 mm, insbesondere 0,4 mm bis 0,9 mm. Aber es sind erfindungsgemäß auch andere Dicken vorgesehen, sofern das Tuch die erforderlich mechanische Stabilität und/oder Flexibilität für die Reinigung gewährleistet.

Die zur Bildung des Tuches eingesetzten Materialien oder Fasern weisen vorzugsweise eine Wasseraufnahmerate von mehr als 60 mm/[10 min] (gemessen mit dem EDANA Test 10.1-72), insbesondere mehr als 80 mm/[10 min] auf. Zusätzlich oder alternativ weisen die zur Bildung des Tuches eingesetzten Materialen oder Fasern vorzugsweise ein Wasseraufnahmevermögen von mehr als 5 g/g (gemessen mit dem EDANA Test 10.1-72), insbesondere mehr als 8 g/g auf.

Die Tücher können oberflächenstrukturiert sein. Diese Prägung kann beispielsweise durch mechanische Prägung mittels Kalanderwalzen oder mittels Wasserstrahlprägung bewerkstelligt werden.

Durch die Strukturierung kann eine gleichmäßige Abfolge von Erhebungen und Senken im Material ausgebildet werden. In Kombination mit der erfindungsgemäßen Tränkungslösungen ermöglicht diese Strukturierung durch ihre Erhebungen sowohl einen besseren Zugang zu Vertiefungen in der menschlichen Haut als auch durch ihre Strukturtäler eine erhöhte Schmutzaufnahmekapazität. Dies führt insgesamt zu einer deutlich verbesserten Reinigungsleistung.

Bezogen auf das nicht oberflächenstrukturierte Tuch kann die Dicke des Tuches mit den durch die Oberflächenstrukturierung erzeugten Erhebungen vorteilhafterweise ungefähr doppelt so groß sein. In bevorzugten Ausführungsformen ist das oberflächenstrukturierte Tuch zwischen 5 % und 50 %, ganz besonders bevorzugt zwischen 10 % und 25 % dicker als das nicht oberflächenstrukturierte Tuch.

Die vorliegende Erfindung betrifft weiterhin die nicht-therapeutische Verwendung eines Zitrusfruchtsaft-haltigen Mittels zum Entfernen von Verschmutzungen organischer und/oder anorganischer Verschmutzungen von der Haut, insbesondere von den Händen. Das erfindungsgemäß verwendete Mittel umfasst
- 5-30 Gew.-%, Zitronensaft oder Zitronensaftkonzentrat, oder einer Mischung von zwei oder mehreren Zitrusfruchtsäften oder Zitrusfruchtsaftkonzentraten aus der Familie der Rutaceae,
- 0,5-10 Gew.-% Phenoxyethanol,
- 2-60 Gew.-%, eines anionischen Tensids oder einer Mischung von zwei oder mehr anionischer Tenside,
- 0,5 bis 3 Gew.-% Toluolsulfonat und/oder Cumolsulfonat und/oder deren Natrium- oder Kaliumsalze,
   und
- ad 100% Wasser,
wobei das anionische Tensid oder die mehreren anionischen Tensids ausgewählt ist/sind aus der Gruppe bestehend aus sekundären Alkansulfonsäuren, sekundären Alkansulfonaten, Alkylethersulfaten, Alkylsulfaten und beliebigen Mischungen davon, und
wobei es sich bei dem anionischen Tensid oder den anionischen Tensiden um die entsprechenden Natrium oder Kaliumsalze handelt, und/oder die primären und/oder sekundären Alkylketten sekundärer Alkansulfonsäuren, sekundärer Alkansulfonaten und Alkylsulfaten jeweils eine Länge von C₁₀-C₂₀, vorzugsweise von C₁₂-C₁₈, aufweisen, und/oder bei Alkylethersulfaten der Ethoxylierungsgrad im Bereich von 3 bis 9 liegt und die Länge der Alkylketten im Bereich von C₈-C₁₈, noch mehr bevorzugt im Bereich von C₁₀-C₁₄ liegt.

Die Verwendung eines solchen Mittels ermöglicht eine schonende, aber effektive Entfernung solcher Verunreinigungen und/oder eine Dekontamination der Haut, insbesondere der Hände.

Bezüglich der chemischen Natur und/oder der Anteile der einzelnen Inhaltsstoffe und bevorzugter Ausführungsformen von diesen wird auf die entsprechende Offenbarung im Zusammenhang mit der Zusammensetzung als solcher Bezug genommen und auf diese verwiesen.

Bei den zu entfernenden Verschmutzung handelt es sich gemäß einer bevorzugten Form der erfindungsgemäßen Verwendung um eine Chlorophyll und/oder andere pflanzliche Farbstoffe enthaltende Verschmutzung und/oder um Pflanzensäfte/-extrakte, sowie um Pflanzenteile, wie Blütenpollen und/oder Pflanzenstäube. Photosynthese betreiben. Sie sind damit in allen grünen Pflanzenteilen enthalten. Der Begriff "farbliche Komponenten" wie im Zusammenhang mit der vorliegenden Erfindung verwendet, meint bzw. umfasst weitere Photosynthesepigmente und gefärbte sekundäre pflanzliche Inhaltsstoffe.

Pflanzensäfte oder Pflanzenextrakte entstehen beispielsweise bei der Ernte, Verpackung, oder Verarbeitung von pflanzlichen Produkten, insbesondere von Tomaten, Paprika und/oder Gurken. Diese Verunreinigungen sind aufgrund ihrer chemischen Zusammensetzung schwer von der Haut zu entfernen. Es hat sich überraschenderweise gezeigt, dass ein wie vorstehend beschriebenes erfindungsgemäßes Zitrusfruchtsaft-haltiges Mittel ganz besonders dazu geeignet ist, solche Verunreinigungen effektiv und schonend von der Haut zu entfernen. Darüber hinaus können durch die Verwendung des Mittels lebensmittelrechtliche und/oder den Schutz von Mitarbeitern betreffenden Aspekten Rechnung getragen werden.

Die erfindungsgemäße Verwendung betrifft daher in einer Ausführungsform der Erfindung die Verwendung einer der vorstehend beschriebenen erfindungsgemäßen Zusammensetzungen zum Entfernen von Chlorophyll und/oder andere pflanzliche Farbstoffe enthaltende Verschmutzungen und/oder zum Entfernen von Pflanzensäften/-extrakten von der Haut. Überraschenderweise bewirkt die hierin beschriebene erfindungsgemäße Zusammensetzung eine mindestens ebenso effektive Reinigung, sie sind aber deutlich weniger belastend für die Haut und damit auch einfacher handhabbar.

Bei den organischen und/oder anorganischen Verschmutzungen, die es von der Haut zu entfernen gilt, kann es sich erfindungsgemäß aber auch, oder zusätzlich zu den Chlorophyll und/oder andere pflanzliche Farbstoffe enthaltenden Verschmutzungen und/oder den Pflanzensäften/-extrakten und/oder den Pflanzenteilen, wie Blütenpollen und/oder Pflanzenstäuben, um unerwünschte Kontaminationen auf der Haut handeln. Bei den Kontaminationen handelt es sich um bakterielle Phytopathogene, pilzliche Phytopathogene, virale Phytopathogene, Düngerrückstände, Herbizide, Bakterizide, Fungizide, Virozide, Keimhemmungsmittel oder beliebige Gemische davon. Dünger, Pflanzenschutzmittel und Keimhemmungsmittel werden beispielsweise im Pflanzenbau großflächig eingesetzt. Die auf den Pflanzenteilen verbleibenden Reste oder abgewaschene Reste können somit zu einer Kontamination der Haut von Mitarbeitern führen.

Auch für diese Art der Verunreinigungen hat sich die erfindungsgemäße Zusammensetzung als besonders wirksam erwiesen, um diese effektiv und schonend von der Haut von kontaminierten Personen zu entfernen.

Die erfindungsgemäße Verwendung betrifft die Reinigung der Haut, insbesondere der Haut der Hände und Arme von den betroffenen Personen.

Die Applikation des Mittels erfolgt bei der erfindungsgemäßen Verwendung entweder direkt als Flüssigkeit oder nach vorhergehender Verdünnung des Mittels. Die Verdünnung des Mittels kann mit Wasser oder einem anderem, geeigneten Lösungsmittel erfolgen. Geeignete Lösungsmittel sind dem Fachmann bekannt. Die Anwendungskonzentration der verdünnten Zusammensetzung beträgt vorzugsweise 1 bis 30 %, vorzugsweise 2 bis 20 %, bezogen auf die Zusammensetzung. Der pH-Wert des verdünnten, gebrauchsfertigen Mittels liegt vorzugsweise im Bereich von 4,75 bis 5,75.

Wie bereits vorstehend erwähnt, kann das gebrauchsfertige Mittel als Einreibeprodukt oder als Hand/-Hautwaschmittel verwendet werden. Für beide Applikationsvarianten beträgt die Einwirkzeit des erfindungsgemäßen Mittels auf der zu reinigenden Hautfläche vorzugsweise mindestens 10 Sekunden, vorzugsweise mindestens 20 Sekunden, noch mehr bevorzugt mindestens 30 Sekunden und ganz besonders bevorzugt mindestens 40 Sekunden. Weiterhin ist bevorzugt, dass die maximale Einwirkzeit des erfindungsgemäßen Mittels auf der Hautfläche 5 Minuten, vorzugsweise 4 Minuten, 3 Minuten oder 2 Minuten beträgt. Die bereits vorstehend erwähnte hohe Verträglichkeit des erfindungsgemäßen Mittels hat sich hierbei als besonders vorteilhaft erwiesen und macht die Einwirkzeiten möglich. Nach der Einwirkzeit wird das Mittel von der Hautfläche abgewaschen (Abwaschprozedere) oder andersartig, beispielsweise mit einem Papiertuch und/oder Textiltuch entfernt (Einreibeprozedur), wobei das Tuch auch ein feucht bzw. mit Wasser getränkt sein kann. Selbstverständlich sind je nach Bedarf auch mehrere aufeinanderfolgende Reinigungen derselben Hautfläche möglich und im Umfang der vorliegenden Erfindung mit umfasst. Das erfindungsgemäße Mittel kann aber auch mit Hilfe der vorstehend beschriebenen feuchten oder getränkten Tücher verwendet werden.

Beschrieben, jedoch nicht beansprucht, ist weiterhin auch ein Verfahren zum Entfernen von organischen und/oder anorganischen Verschmutzungen von der Haut, vorzugsweise von den Händen, bereit, wobei das Verfahren die Schritte umfasst von:
- Bereitstellen einer Zitrusfruchtsaft-haltigen Zusammensetzung, welche 5-30 Gew.-% Zitrusfruchtsaft oder eines Zitrusfruchtsaftkonzentrats oder einer Mischung von zwei oder mehreren solcher Zitrusfruchtsäfte oder Zitrusfruchtsaftkonzentraten aus der Familie der Rutaceae, 0,5-10 Gew.-% Phenoxyethanol, 2-60 Gew.-% eines anionischen Tensids oder einer Mischung von zwei oder mehr anionischer Tenside, 0,5 bis 3 Gew.-% Toluolsulfonat und/oder Cumolsulfonat und/oder deren Natrium- oder Kaliumsalze und Wasser umfasst, wobei das anionische Tensid oder die mehreren anionischen Tensids ausgewählt ist/sind aus der Gruppe bestehend aus sekundären Alkansulfonsäuren, sekundären Alkansulfonaten, Alkylethersulfaten, Alkylsulfaten und beliebigen Mischungen davon, und
   wobei es sich bei dem anionischen Tensid oder den anionischen Tensiden um die entsprechenden Natrium oder Kaliumsalze handelt, und/oder die primären und/oder sekundären Alkylketten sekundärer Alkansulfonsäuren, sekundärer Alkansulfonaten und Alkylsulfaten jeweils eine Länge von C₁₀-C₂₀, vorzugsweise von C₁₂-C₁₈, aufweisen, und/oder bei Alkylethersulfaten der Ethoxylierungsgrad im Bereich von 3 bis 9 liegt und die Länge der Alkylketten im Bereich von C₈-C₁₈, noch mehr bevorzugt im Bereich von C₁₀-C₁₄ liegt
   - Aufbringen der bereitgestellten Zitrusfruchtsaft-haltigen Zusammensetzung auf eine mit organischen und/oder anorganischen Verschmutzungen verschmutzten Hautfläche, und
   - Reinigen der Hautfläche.
   - Das Aufbringen / die Applikation der Zusammensetzung erfolgt im erfindungsgemäßen Verfahren entweder direkt als Flüssigkeit oder nach vorheriger Verdünnung, beispielsweise mit Wasser. Durch eine mechanische Einwirkung kann nachträglich eine Schaumbildung ausgelöst werden.

Das Reinigen der Hautfläche, beispielsweise der Hände und Arme, kann über ein Abwaschprozedere erfolgen, wie es auch der normalen Körperhygiene, beispielsweise beim Händewaschen mit einer handelsüblichen Seife, durchgeführt wird. Alternativ, ist aber auch eine Einreibeprozedur denkbar. Dort wird das Mittel auf der zu reinigenden Hautfläche, beispielsweise den Händen und Armen ohne Wasser verteilt und eingerieben, und anschließend mit einem Papiertuch und/oder Textiltuch, wobei das Tuch auch feucht bzw. mit Wasser getränkt sein kann, entfernt. Alternativ kann das Aufbringen / die Applikation der Zusammensetzung mittels des vorstehend beschriebenen feuchten oder getränkten Tuchs erfolgen.

Bezüglich der Einwirkzeit des Mittels auf der zu reinigenden Hautfläche wird - für die beiden Applikationsformen Einreibeprozedur oder Abwaschprozedere - auf die entsprechenden Ausführungen im Zusammenfang mit der erfindungsgemäßen Verwendung Bezug genommen. Die Zeiten gelten für die erfindungsgemäße Verwendung und das erfindungsgemäße Verfahren gleichermaßen.

Nach der Einwirkzeit wird das Mittel von der Hautfläche abgewaschen oder andersartig entfernt. Der Schritt des Abwaschens oder Entfernen des Mittels kann beim Einsatz der feuchten oder getränkten Tücher entfallen. Selbstverständlich sind je nach Bedarf auch mehrere aufeinanderfolgende Reinigungen möglich und im Umfang der vorliegenden Erfindung mit umfasst. Das erfindungsgemäße Verfahren kann also die weiteren Schritte eines Einwirkenlassens der applizierten Zusammensetzung, vorzugsweise mit den vorstehend genannten Einwirkzeiten, und/oder den Schritt eines Abwaschen oder andersartigen Entfernens der applizierten Zusammensetzung, gegebenenfalls nach deren Einwirkung, umfassen.

Es ist erfindungsgemäß ebenso vorgesehen, dass die Zusammensetzung vor deren Applikation als Lösung in einem vorgeschalteten Verfahrensschritt auf eine gewünschte Anwendungskonzentration verdünnt wird. Die Verdünnung der Zusammensetzung kann mit Wasser oder einem anderem, geeigneten Lösungsmittel erfolgen. Geeignete Lösungsmittel sind dem Fachmann bekannt. Die Anwendungskonzentration der verdünnten Zusammensetzung beträgt dann vorzugsweise 1 bis 30 Gew.-%, vorzugsweise 2 bis 20 Gew.-% der ursprünglichen Zusammensetzung. Die Verdünnung der Zusammensetzung zur Gebrauchslösung erfolgt vorzugsweise mit Wasser. Das beschriebene jedoch nicht beanspruchte Verfahren kann also den weiteren, und der Applikation vorgeschalteten Schritt eines Verdünnens der zu applizierenden Zusammensetzung, vorzugsweise auf die vorstehend genannten Anwendungs-konzentrationen, umfassen.

In einer bevorzugten Ausführungsform umfasst das beschriebene jedoch nicht beanspruchte Verfahren somit die Schritte
- Bereitstellen einer Zitrusfruchtsaft-haltigen Zusammensetzung, die wie vorstehend beschrieben zusammengesetzt ist,
- gegebenenfalls Verdünnen der bereitgestellten Zitrusfruchtsaft-haltigen Zusammensetzung auf eine Anwendungskonzentration von vorzugsweise 1 bis 30Gew.-%, vorzugsweise 2 bis 20 Gew.-%, vorzugsweise mit Wasser und/oder einem anderen geeigneten Lösungsmittel,
- Applizierens der, gegebenenfalls verdünnten, Zusammensetzung auf eine zu reinigende Hautfläche, vorzugsweise die Hände und/oder Arme,
- Einwirkenlassen der applizierten Zusammensetzung, vorzugsweise für einen Zeitraum von 10 Sekunden bis 5 Minuten, und
- Gegebenenfalls Abwaschen oder Entfernen der applizierten Zusammensetzung.

Bezüglich bevorzugter Ausführungsformen des im beschriebenen jedoch nicht beanspruchten Verfahren eingesetzten Mittels wird auf die vorstehenden Ausführungen zur Zusammensetzung als solcher verwiesen und Bezug genommen.

Gemäß einer Ausführungsform des beschriebenen jedoch nicht beanspruchten Verfahrens wird die Zitrusfruchtsaft-haltige Zusammensetzung zum Entfernen von Chlorophyll und/oder andere pflanzliche Farbstoffe enthaltenden Verschmutzungen und/oder zum Entfernen von Pflanzensäften/- extrakten verwendet.

In einer weiteren beschriebenen jedoch nicht beanspruchten Ausführungsform wird ein Verfahren zum Entfernen von Kontaminationen auf der Haut bereitgestellt, wobei die Kontaminationen vorzugsweise ausgewählt sind aus der Gruppe, bestehend aus bakteriellen Phytopathogenen, pilzlichen Phytopathogenen, viralen Phytopathogenen, Düngerrückständen, Herbiziden, Bakteriziden, Fungiziden, Viroziden, Keimhemmungsmitteln und beliebige Gemische davon.

Anhaftungen von Pflanzensäften/-extrakten auf der Haut können nicht nur aus rein kosmetischen Gründen unerwünscht sein, sondern die Pflanzensäfte/-extrakte können unter Umständen auch zu Hautreizungen und/oder allergischen Reaktionen bei den betroffenen Personen führen. Rückstände oder Anhaftungen von Dünger, Herbiziden, Bakteriziden, Fungiziden, Viroziden, Keimhemmungsmitteln und/oder Pathogenen an beispielsweise den Händen von Mitarbeitern können unter anderem in Meristem- oder Pflanzenzuchtbetrieben und pflanzenproduzierenden Betrieben nicht nur deshalb problematisch sein, weil sie so im Betrieb unkontrolliert auf andere Kulturen verteilt werden können, sondern auch aus einem gesundheitlichen Aspekt zum Schutz der Mitarbeiter. Eine Inkorporation dieser Substanzen ist zu vermeiden.

Gegenstand der vorliegenden Erfindung ist ferner eine Zitrusfruchtsaft-haltige Zusammensetzung, die wie vorstehend beschrieben zusammengesetzt ist, zur Verwendung in einem Verfahren zur hygienischen Reinigung und Dekontamination der Haut und/ Hände eines Benutzers. Dieser Gegenstand betrifft also eine Zusammensetzung zur therapeutischen oder prophylaktischen Verwendung bei der Entfernung von Verschmutzungen von der Haut, wobei es sich bei den Verschmutzungen um bakterielle Phytopathogene, pilzliche Phytopathogene oder virale Phytopathogene handelt.

Die Haut, beispielsweise die Hände, wird/werden mit Hilfe der Zusammensetzung von organischen und/oder anorganischen Verschmutzungen befreit, und damit aus den vorstehend genannten Gründen eine dem Schutz der Gesundheit der Menschen, die solchen Verschmutzungen ausgesetzt sind, vorbeugende Maßnahme durchgeführt.

Eine Ausführungsform betrifft die nicht-therapeutische Verwendung der Zusammensetzung in einem Verfahren zur Reinigung der Haut und/ Hände von Chlorophyll und/oder andere pflanzliche Farbstoffe enthaltenden Verschmutzungen und/oder zum Entfernen von Pflanzensäften/-extrakten.

Eine weitere Ausführungsform betrifft die nicht-therapeutische Verwendung der Zusammensetzung in einem Verfahren zur Reinigung und Dekontamination der Haut und/ Hände von Kontaminationen, wobei die Kontaminationen vorzugsweise ausgewählt sind aus der Gruppe, bestehend aus bakteriellen Phytopathogenen, pilzlichen Phytopathogenen, viralen Phytopathogenen, Düngerrückständen, Herbiziden, Bakteriziden, Fungiziden, Virozide, Keimhemmungsmitteln und beliebige Gemische davon.

Ebenso betrifft die vorliegende Erfindung eine Zitrusfruchtsaft-haltigen Zusammensetzung, die wie vorstehend beschrieben zusammengesetzt ist, zur Verwendung in einem Verfahren zur Reinigung der Haut und/ Hände, um Hautreizungen und/oder allergischen Reaktionen vorzubeugen, die durch Pflanzensäfte/-extrakte verursacht werden können. Weiterhin ist erfindungsgemäß umfasst eine Zitrusfruchtsaft-haltigen Zusammensetzung, die wie vorstehend beschrieben zusammengesetzt ist, zur Verwendung in einem Verfahren zur Vermeidung einer Inkorporation von Kontaminationen, wobei die Kontaminationen vorzugsweise ausgewählt sind aus der Gruppe, bestehend aus bakteriellen Phytopathogenen, pilzlichen Phytopathogenen, viralen Phytopathogenen, Düngerrückständen, Herbiziden, Bakteriziden, Fungiziden, Viroziden, Keimhemmungsmitteln und beliebigen Gemische davon.

Bezüglich bevorzugter Ausführungsformen der eingesetzten Zusammensetzung, die Art und die Art und Weise der Applikation der Zusammensetzung wird auf die vorstehenden Ausführungen zur Zusammensetzung als solcher und zur erfindungsgemäßen Verwendung verwiesen und Bezug genommen.

Die nachfolgenden Beispiele erläutern die Erfindung, ohne sie jedoch darauf einzuschränken; alle Angaben sind, sofern nicht anders angegeben, in Gew.-%.

### Beispiele

Nachfolgende Beispiele und Erläuterungen zur Zusammensetzung sollen die erfindungsgemäßen Gegenstände verdeutlichen.

Der synergistische Effekt zwischen Zitrusfruchtsäften, Phenoxyethanol und den anionischen Tensiden lässt sich besonders anschaulich am Beispiel von Orangen- Pampelmusen- und Zitronensaft darstellen.

Frisch gepresster Saft reifer Zitronen, Orangen und Pampelmusen (Citrus grandis) wurde durch Filtration vom Fruchtfleisch (Pulpe) befreit. Der Gesamtsäuregehalt der Säfte, deren saure Komponenten sich überwiegend aus Zitronensäure und nur in sehr geringem Maße aus Iso-Zitronensäure, Ascorbinsäure und weiteren sauren Bestandteilen zusammensetzen, wurde durch Titration aus dem Filtrat bestimmt.

Die Titrationsergebnisse wurden in Zitronensäure umgerechnet, wodurch sich in dem filtrierten Saft eine mittlere theoretische Konzentration von 4,8 Gew.-%, bezogen auf 3 bis 8 Gew.-% Zitronensäure, ergab. Der pH-Wert des Zitronensaftes wurde zu pH 2,34 ermittelt, der Pampelmusensaft zu 2,68 und der des Orangensaftes zu pH 3,05.

Die analytische Klassifizierung der Säfte, diente der Standardisierung zur Wiederholbarkeit der Untersuchungen.

Das Serum der Säfte bestand aus klaren, leicht bis intensiv gelben, geruchsarmen Flüssigkeiten, die zur Herstellung der erfindungsgemäßen Mittel mit Phenoxyethanol und Tensiden vermischt wurden und als gebrauchsfertige Lösungen zur Reinigung der Hände eingesetzt wurden.

### Beispiel 1: Zitronensaft-haltiges Mittel

| Komponenten | Gewichtsteile (Gew./Gew.) |
|---|---|
| Alkyl(C₁₂-C₁₈)-sulfonat-Na | 18,00 |
| Na-(C₁₃)-Alkylethersulfonat | 20,00 |
| Cumolsulfonat-K | 2,00 |
| Phenoxyethanol | 4,00 |
| Zitronensaft | 15,00 |
| Wasser | 41,00 |

### Beispiel 2: Zitronensaft-haltiges Mittel

| Komponenten | Gewichtsteile (Gew./Gew.) |
|---|---|
| Sec.-Alkyl(C₁₂-C₁₈)-sulfonat -Na | 10,00 |
| Na-dodecylethersulfat | 10,00 |
| Toluolsulfonat-K | 3,00 |
| Phenoxyethanol | 2,00 |
| Zitronensaft | 15,00 |
| Wasser | 60,00 |

### Beispiel 3: Zitronensaft-haltiges Mittel

| Komponenten | Gewichtsteile (Gew./Gew.) |
|---|---|
| Sec.-Alkyl(C₁₂-C₁₈)-sulfonat -Na | 3,00 |
| Na-dodecylethersulfat | 3,00 |
| Toluolsulfonat-K | 2,00 |
| Phenoxyethanol | 2,00 |
| Zitronensaft | 10,00 |
| Wasser permutiert | 80,00 |

### Beispiel 4: Orangen- oder Pampelmusensaft-haltiges Reinigungsmittel

Anstelle des Zitronenfruchtsafts können die Beispiele 1-3 auch mit Orangenfruchtsaft (nicht erfindungsgemäß), Pampelmusenfruchtsaft (nicht erfindungsgemäß) oder einer Citrusfruchtsaftmischung aus 50 Gew.-% Zitronenfrucht-saft und 50 Gew.-% Orangen-oder Pampelmusenfruchtsaft formuliert sein.

Bei der Herstellung der Beispielformulierungen 1 bis 4 geht man so vor, dass zunächst das Wasser, vorzugsweise gereinigtes Wasser, vorgelegt wird und darin dann der Zitrusfruchtsaft gelöst werden. Dieser Lösung werden dann die Tenside und die weiteren Komponenten hinzugegeben, evtl. weiteres Wasser ad 100 Gew.-% ergänzt und verrührt. Es entsteht eine klare Flüssigkeit.

Es hat sich gezeigt, dass durch die beispielhaften erfindungsgemäßen Zusammensetzungen Chlorophyll-haltige Verschmutzungen durch Händewaschen mit einer Einwirkzeit von 30 Sekunden effektiv und schonend von der Haut entfernt werden konnten. Der Epikutantest zur Prüfung der hautirritierenden Wirkung zeigte eine gute Hautverträglichkeit der erfindungsgemäßen Prüflösungen.

Vergleichbare positive Resultate konnten bei der Entfernung von Düngerrückständen erzielt werden. Auch hier war die Reinigung sehr effektiv und schonend.

## Patentansprüche

1. Zusammensetzung zur Reinigung von Haut und/oder Händen, umfassend:
- 5-30 Gew.-% Zitronensaft oder Zitronensaftkonzentrat, oder einer Mischung von zwei oder mehreren Zitrusfruchtsäften oder Zitrusfruchtsaftkonzentraten aus der Familie der Rutaceae,
- 0,5-10 Gew.-% Phenoxyethanol,
- 2-60 Gew.-% eines anionischen Tensids oder einer Mischung von zwei oder mehr anionischer Tenside,
- 0,5 bis 3 Gew.-% Toluolsulfonat und/oder Cumolsulfonat und/oder deren Natrium- oder Kaliumsalze,
und
- ad 100 Gew.-% Wasser,
wobei das anionische Tensid oder die mehreren anionischen Tenside ausgewählt ist/sind aus der Gruppe bestehend aus sekundären Alkansulfonsäuren, sekundären Alkansulfonaten, Alkylethersulfaten, Alkylsulfaten und beliebigen Mischungen davon, und
wobei es sich bei dem anionischen Tensid oder den anionischen Tensiden um die entsprechenden Natrium oder Kaliumsalze handelt, und/oder die primären und/oder sekundären Alkylketten sekundärer Alkansulfonsäuren, sekundärer Alkansulfonaten und Alkylsulfaten jeweils eine Länge von C₁₀-C₂₀, vorzugsweise von C₁₂-C₁₈, aufweisen, und/oder bei Alkylethersulfaten der Ethoxylierungsgrad im Bereich von 3 bis 9 liegt und die Länge der Alkylketten im Bereich von C₈-C₁₈, noch mehr bevorzugt im Bereich von C₁₀-C₁₄ liegt.

2. Zusammensetzung nach Anspruch 1, wobei der Zitrusfruchtsaft bzw. das Zitrusfruchtsaftkonzentrat ausgewählt ist aus der Gruppe, bestehend aus Zitronensaft, Orangensaft, Bitterorangensaft, Pampelmusensaft, Grapefruitsaft, Mandarinensaft, Limettensaft, Yuzusaft, Kabosusaft, Sudachisaft, Shiikuwashasaft, Tangerinensaft, Pumellensaft, Mexican lime-Saft, Kumquatsaft oder Gemischen daraus.

3. Zusammensetzung nach einem der vorhergehenden Ansprüche, wobei das Gewichtsverhältnis von Zitrusfruchtsaft oder Zitrusfruchtsaftkonzentrat zu Phenoxyethanol 1:0,1 bis 1:0,8, vorzugsweise 1:0,2 bis 1:0,6 beträgt.

4. Zusammensetzung nach einem der vorhergehenden Ansprüche, weiterhin umfassend ein nichtionisches Tensid, vorzugsweise ein Fettalkoholethoxylat, oder einer Mischung von zwei oder mehr nichtionischer Tenside, wobei die Mischung vorzugsweise mindestens ein Fettalkoholethoxylat umfasst, und/oder wobei der Anteil des nichtionischen Tensids oder der nichtionischen Tensidmischung, bezogen auf die Gesamtzusammensetzung, 0,1-5 Gew.-% beträgt.

5. Zusammensetzung nach einem der vorhergehenden Ansprüche, weiterhin umfassend ein oder mehrere Verdickungsmittel, vorzugsweise mit einem Gesamtanteil von 0,05 bis 1 Gew.-%.

6. Zusammensetzung nach einem der vorhergehenden Ansprüche, wobei die Zusammensetzung frei von Geruchsstoffen und/oder Parfüm und/oder Alkoholen, vorzugsweise primären C₁-C₅-Alkoholen ist.

7. Zusammensetzung nach einem der vorhergehenden Ansprüche, wobei die Zusammensetzung als eine gebrauchsfertige Wirkstofflösung formuliert ist, wobei es sich bei der gebrauchsfertigen Lösung vorzugsweise um entweder ein Einreibeprodukt oder um ein Hand/Hautwaschmittel handelt.

8. Feuchtes Tuch, umfassend ein Tuch aus einem Zellstoffmaterial auf der Basis von Cellulose, einem Gewebe und/oder einem Vliesgewebe, wobei das Tuch mit der Zusammensetzung nach einem der Ansprüche 1 bis 7 als Tränkungslösung befeuchtet ist.

9. Feuchtes Tuch nach Anspruch 8, wobei das Gewichtsverhältnis des ungetränkten Tuchs zu der Tränkungslösung aus dem Bereich von 1 : 1 bis 1 : 5 gewählt wird.

10. Nicht-therapeutische Verwendung der Zusammensetzung nach einem der Ansprüche 1 bis 7 zur Entfernung von organischer und/oder anorganischer Verschmutzungen von der Haut, vorzugsweise von den Händen.

11. Nicht-therapeutische Verwendung nach Anspruch 10, wobei es sich bei der zu entfernenden Verschmutzung um eine Chlorophyll und/oder andere pflanzliche Farbstoffe enthaltende Verschmutzung und/oder um Pflanzensäfte/-extrakte und/oder andere Pflanzenteile, wie Blütenpollen und/oder Pflanzenstäube, handelt.

12. Nicht-therapeutische Verwendung nach Anspruch 10, wobei es sich bei der zu entfernenden Verschmutzung um Kontaminationen auf der Haut handelt, wobei die Kontaminationen vorzugsweise ausgewählt sind aus der Gruppe, bestehend aus bakteriellen Phytopathogenen, pilzlichen Phytopathogenen, viralen Phytopathogenen, Düngerrückständen, Herbiziden, Bakteriziden, Fungiziden, Viroziden, Keimhemmungsmitteln und beliebigen Gemische davon.

13. Nicht-therapeutische Verwendung nach einem der Ansprüche 10 bis 12, wobei die Zusammensetzung als verdünnte, wässerige Lösung angewendet wird, wobei die Zusammensetzung vorzugsweise durch Verdünnen mit Wasser auf Wirkstoffkonzentrationen im Bereich von 0,5 bis 50 %, vorzugsweise im Bereich von 1 bis 30 %, eingestellt ist.

14. Nicht-therapeutische Verwendung nach einem der Ansprüche 10 bis 12, wobei die Zusammensetzung als gebrauchsfertiges Einreibeprodukt oder als ein Hand/Hautwaschmittel verwendet wird.

15. Nicht-therapeutische Verwendung eines feuchten Tuchs nach Anspruch 8 oder Anspruch 9 zur Entfernung von organischer und/oder anorganischer Verschmutzungen von der Haut, vorzugsweise von den Händen, wobei es sich bei der Verschmutzung vorzugsweise um Kontaminationen auf der Haut handelt, die ausgewählt sind aus der Gruppe, bestehend aus bakteriellen Phytopathogenen, pilzlichen Phytopathogenen, viralen Phyto-pathogenen, Düngerrückständen, Herbiziden, Bakteriziden, Fungiziden, Viroziden, Keimhemmungsmitteln und beliebigen Gemische davon, und/oder wobei es sich bei den der zu entfernenden Verschmutzung um eine Chlorophyll und/oder andere pflanzliche Farbstoffe enthaltende Verschmutzung und/oder um Pflanzensäfte/-extrakte und/oder andere Pflanzenteile, wie Blütenpollen und/oder Pflanzenstäube, handelt.

16. Zusammensetzung nach einem der Ansprüche 1 bis 7 zur therapeutischen oder prophylaktischen Verwendung bei der Entfernung von Verschmutzungen von der Haut, wobei es sich bei den Verschmutzungen um bakterielle Phytopathogene, pilzliche Phytopathogene oder virale Phytopathogene handelt.

17. Zusammensetzung zur Verwendung nach Anspruch 16 , wobei die Zusammensetzung als verdünnte, wässerige Lösung angewendet wird, wobei die Zusammensetzung vorzugsweise durch Verdünnen mit Wasser auf Wirkstoffkonzentrationen im Bereich von 0,5 bis 50 %, vorzugsweise im Bereich von 1 bis 30 %, eingestellt ist.

18. Zusammensetzung zur Verwendung nach einem der Ansprüche 16 bis 17, wobei die Zusammensetzung als gebrauchsfertiges Einreibeprodukt oder als ein Hand/Hautwaschmittel verwendet wird.

19. Feuchtes Tuchs nach Anspruch 8 oder Anspruch 9 zur therapeutischen oder prophylaktischen Verwendung bei der Entfernung von Verschmutzungen von der Haut, wobei es sich bei den Verschmutzungen um bakterielle Phytopathogene, pilzliche Phytopathogene oder virale Phytopathogene handelt.

## Claims

1. A composition for cleaning skin and/or hands comprising:
- 5-30 % by weight of lemon juice or lemon juice concentrate, or a mixture of two or more citrus juices or citrus juice concentrates from the family of Rutaceae,
- 0.5-10 % by weight of phenoxyethanol,
- 2-60 % by weight of an anionic surfactant or a mixture of two or more anionic surfactants,
- 0.5-3% by weight of toluene sulphonate and/or cumene sulphonate their sodium or potassium salts
and
- water to 100 % by weight,
wherein the anionic surfactant or the plurality of anionic surfactants is/are selected from the group consisting of secondary alkane sulphonic acids, secondary alkane sulphonates, alkyl ether sulphates, alkyl sulphates and any mixtures thereof, and
wherein the anionic surfactant or anionic surfactants are included as the corresponding sodium or potassium salts, and/or the primary and/or secondary alkyl chains of the secondary alkane sulphonic acids, secondary alkane sulphonates, and alkyl sulphates each have a length of C₁₀-C₂₀, preferably of C₁₂-C₁₈, and/or in the case of the alkyl ether sulphates the degree of ethoxylation is in the range from 3 to 9 and the length of the alkyl chains is in the range from C₈-C₁₈, even more preferably in the range from C₁₀-C₁₄.

2. The composition according to claim 1, wherein the citrus fruit juice or citrus fruit juice concentrate is selected from the group consisting of lemon juice, orange juice, bitter orange juice, pomelo juice, grapefruit juice, mandarin juice, lime juice, yuzu juice, kabosu juice, sudachi juice, shikuwash juice, tangerine juice, pumelon juice, Mexican lime juice, kumquat juice or mixtures thereof.

3. The composition according to one of the preceding claims, wherein the weight ratio of lemon juice or lemon juice concentrate to phenoxyethanol is from 1:0.1 to 1:0.8, preferably from 1:0.2 to 1:0.6.

4. The composition according to any one of the preceding claims, further comprising a non-ionic surfactant, preferably a fatty alcohol ethoxylate, or a mixture of two or more non-ionic surfactants, wherein the mixture preferably comprises at least one fatty alcohol ethoxylate, and/or wherein the proportion of the non-ionic surfactant or the non-ionic surfactant mixture, based on the total composition, is 0.1-5 % by weight .

5. The composition according to any one of the preceding claims, further comprising one or more thickening agents, preferably with a total content of from 0.05 to 1% by weight.

6. The composition according to any one of the preceding claims, wherein the composition is free from odorants and/or perfume and/or alcohols, preferably primary C₁ -C₅ alcohols.

7. The composition according to any one of the preceding claims, wherein the composition is formulated as a ready-to-use active ingredient solution, wherein the ready-to-use solution preferably is either a rub-in product or a hand/skin wash.

8. A wet cloth comprising a cloth of a cellulose-based pulp material, a woven fabric and/or a non-woven fabric, wherein the cloth is wetted with the composition of any one of claims 1 to 7 as an impregnating solution.

9. A wet cloth according to claim 8, wherein the weight ratio of the unimpregnated cloth to the impregnating solution is selected from the range of 1 : 1 to 1 : 5.

10. Non-therapeutical use of the composition according to any one of claims 1 to 7 for removing organic and/or inorganic soiling from the skin, preferably from the hands.

11. Non-therapeutical use according to claim 10, wherein the contaminant to be removed is a contaminant containing chlorophyll and/or other plant dyes and/or plant juices/extracts and/or other plant parts, such as flower pollen and/or plant dusts.

12. Non-therapeutical use according to claim 10, wherein the contamination to be removed is contaminants on the skin, said contaminants preferably being selected from the group consisting of bacterial phytopathogens, fungal phytopathogens, viral phytopathogens, fertilizer residues, herbicides, bactericides, fungicides, virucides, germicides and any mixtures thereof.

13. Non-therapeutical use according to any one of claims 10 to 12, wherein the composition is applied as a diluted aqueous solution, the composition preferably being adjusted by dilution with water to active ingredient concentrations in the range from 0.5 to 50%, preferably in the range from 1 to 30%.

14. Non-therapeutical use according to any one of claims 10 to 12, wherein the composition is used as a ready-to-use rub-in product or as a hand/skin wash.

15. Non-therapeutical use of a wet cloth according to claim 8 or claim 9 for removing organic and/or inorganic soiling from the skin, preferably from the hands, wherein the soiling is preferably contaminants on the skin selected from the group consisting of bacterial phytopathogens, fungal phytopathogens, viral phytopathogens, fertilizer residues, herbicides, bactericides, fungicides, virucides, germicides and any mixtures thereof, and/or wherein the contaminants to be removed are contaminants containing chlorophyll and/or other plant dyes and/or plant juices/extracts and/or other plant parts, such as pollen and/or plant dusts.

16. The composition according to any of claims 1 to 7 for therapeutic or prophylactic use in removing soiling from the skin,
wherein the soiling is bacterial phytopathogens, fungal phytopathogens or viral phytopathogens.

17. The composition for use according to claim 16, wherein the composition is used as a diluted aqueous solution, wherein the composition is preferably adjusted to active ingredient concentrations in the range of 0.5 to 50%, preferably in the range of 1 to 30%, by dilution with water.

18. The composition for use according to any of claims 16 to 17, wherein the composition is used as a ready-to-use rubbing product or as a hand/skin wash.

19. The wet cloth according to claim 8 or claim 9 for therapeutic or prophylactic use in removing soiling from the skin, wherein the soiling is bacterial phytopathogens, fungal phytopathogens or viral phytopathogens.

## Revendications

1. Composition destinée au nettoyage de la peau et/ou des mains, comprenant:
- 5 à 30 % en poids de jus de citron ou concentré de jus de citron, ou d'un mélange de deux ou plusieurs jus d'agrumes ou concentrés de jus d'agrumes appartenant à la famille des Rutacées,
- 0,5 à 10 % en poids de phénoxyéthanol,
- 2 à 60 % d'un tensioactif anionique ou d'un mélange d'au moins deux ou tensioactifs anioniques,
- 0,5 à 3 % en poids de toluènesulfonate et/ou cumènesulfonate et/ou de leurs sels de sodium ou de potassium,
et
- le complément à 100 % en poids étant de l'eau,
le tensioactif anionique ou les plusieurs tensioactifs anioniques étant choisi(s) dans le groupe constitué d'acides alcanesulfoniques secondaires, d'alcanesulfonates secondaires, de sulfates d'alkyléther, de sulfates d'alkyle et de tout mélange de ceux-ci,
et
le tensioactif anionique ou les tensioactifs anioniques correspondant à leurs sels de sodium ou de potassium, et/ou les chaînes alkyle primaires ou secondaires d'acides alcanesulfoniques secondaires, d'alcanesulfonates secondaires et de sulfates d'alkyle présentant chacune des longueurs de C₁₀ à C₂₀, préférentiellement de C₁₂ à C₁₈, et/ou le degré d'éthoxylation des sulfates d'alkyléther étant compris entre 3 et 9, et la longueur des chaînes d'alkyle étant comprise entre C₈ et C₁₈, de manière plus préférée comprise entre C₁₀ et C₁₄.

2. Composition selon la revendication 1, le jus d'agrumes ou le concentré de jus d'agrumes étant choisi dans le groupe constitué de jus de citron, jus d'orange, jus d'orange amère, jus de pamplemousse, jus de pomelo, jus de mandarine, jus de citron vert, jus de yuzu, jus de kabosu, jus de sudachi, jus de Citrus depressa, jus de tangerine, jus d'autres hybrides de type Citrus x paradisi, jus de lime acide, jus de kumquat ou de leurs mélanges.

3. Composition selon l'une des revendications précédentes, le rapport pondéral entre le jus d'agrumes ou le concentré de jus d'agrumes et le phénoxyéthanol étant compris entre 1 : 0,1 et 1 : 0,8, préférentiellement entre 1 : 0,2 et 1 : 0,6.

4. Composition selon l'une des revendications précédentes, comprenant en outre un tensioactif non-ionique, s'agissant préférentiellement d'un éthoxylate d'alcool gras, ou un mélange d'au moins deux ou tensioactifs non-ioniques, ledit mélange comprenant de préférence au moins un éthoxylate d'alcool gras, et/ou la proportion du tensioactif non-ionique ou du mélange de tensioactifs non-ioniques étant comprise, par rapport à la totalité de la composition, entre 0,1 et 5 % en poids.

5. Composition selon l'une ou plusieurs des revendications précédentes, comprenant en outre un ou plusieurs épaississants, leur proportion totale étant préférentiellement comprise entre 0,05 et 1 % en poids.

6. Composition selon l'une des revendications précédentes, ladite composition étant exempte de composés odorants et/ou de parfums et/ou d'alcools, préférentiellement d'alcools primaires en C₁ à C₅.

7. Composition selon l'une des revendications précédentes, ladite composition étant formulée en tant que solution de principes actifs prête à l'emploi, la solution prête à l'emploi étant de préférence soit une produit à étaler et/ou un nettoyant pour les mains / la peau.

8. Lingette humide, comprenant une lingette fabriquée à partir d'un matériau cellulosique à base de cellulose, d'un tissu ou d'un non-tissé, ladite lingette étant humidifiée avec la composition selon l'une des revendications 1 à 7 en tant que solution d'imprégnation.

9. Lingette humide selon la revendication 8, le rapport pondéral entre la lingette non-imprégnée et la solution d'imprégnation étant choisi de manière à être compris entre 1 : 1 et 1 : 5.

10. Utilisation non-thérapeutique de la composition selon l'une des revendications 1 à 7 pour enlever des salissures organiques et/ou inorganiques présentes sur la peau, préférentiellement sur les mains.

11. Utilisation non-thérapeutique selon la revendication 10, la salissure devant être enlevée correspondant à une salissure contenant de la chlorophylle et/ou d'autres colorants végétaux et/ou à des jus/extraits de plantes et/ou à d'autres parties de plantes telles que des pollens de fleurs et/ou des particules libérées par des plantes.

12. Utilisation non-thérapeutique selon la revendication 10, la salissure devant être enlevé correspondant à des contaminations présentes sur la peau, lesdites contaminations étant préférentiellement choisies dans le groupe constitué de phytopathogènes bactériens, de phytopathogènes cryptogamiques, de phytopathogènes viraux, de résidus d'engrais, d'herbicides, de bactéricides, de fongicides, de virucides, 'inhibiteurs de germination et de tout mélange de ceux-ci.

13. Utilisation non-thérapeutique selon l'une des revendications 10 à 12, ladite composition étant mise en œuvre en tant que solution aqueuse diluée, ladite composition étant préférentiellement préparée, par dilution avec de l'eau, de manière à ce que sa concentration en principes actifs soit comprise entre 0,5 et 50 %, de préférence entre 1 et 30 %.

14. Utilisation non-thérapeutique selon l'une des revendications 10 à 12, ladite composition étant utilisée en tant que produit à étaler prêt à l'emploi ou en tant que nettoyant pour les mains / la peau.

15. Utilisation non-thérapeutique d'une lingette humide selon la revendication 8 ou la revendication 9 pour enlever des salissures organiques et/ou inorganiques présentes sur la peau, de préférence sur les mains, ladite salissure correspondant préférentiellement des contaminations présentes sur la peau, lesquelles sont choisies dans le groupe constitué de phytopathogènes bactériens, de phytopathogènes cryptogamiques, de phytopathogènes viraux, de résidus d'engrais, d'herbicides, de bactéricides, de fongicides, de virucides, d'inhibiteurs de germination et de tout mélange de ceux-ci, et/ou la salissure devant être enlevée correspondant à une salissure contenant de la chlorophylle et/ou d'autres colorants végétaux et/ou à des jus/extraits de plantes et/ou à d'autres parties de plantes telles que des pollens de fleurs et/ou des particules libérées par des plantes.

16. Composition selon l'une des revendications 1 à 7, pour une utilisation thérapeutique ou prophylactique lors qu'il s'agit d'enlever des salissures présentes sur la peau, lesdites salissures étant des phytopathogènes bactériens, des phytopathogènes cryptogamiques ou des phytopathogènes viraux.

17. Composition destinée à une utilisation selon la revendication 16, ladite composition étant mise en œuvre en tant que solution aqueuse diluée, ladite composition étant préférentiellement préparée, par dilution avec de l'eau, de manière à ce que sa concentration en principes actifs soit comprise entre 0,5 et 50 %, de préférence entre 1 et 30 %.

18. Composition destinée à une utilisation selon l'une des revendications 16 à 17, ladite composition étant utilisée en tant que produit à étaler prêt à l'emploi ou en tant que nettoyant pour les mains / la peau.

19. Lingette humide selon la revendication 8 ou la revendication 9, destinée à une utilisation thérapeutique ou prophylactique lors qu'il s'agit d'enlever des salissures présentes sur la peau, lesdites salissures étant des phytopathogènes bactériens, des phytopathogènes cryptogamiques ou des phytopathogènes viraux.
